(19) **Europäisches Patentamt**
**European Patent Office**
**Office européen des brevets**

(11) **EP 3 714 892 B1**

(12) **EUROPEAN PATENT SPECIFICATION**

(45) Date of publication and mention
of the grant of the patent:
**25.06.2025 Bulletin 2025/26**

(51) International Patent Classification (IPC):
**A61K 35/28** (2015.01)     **A61K 35/12** (2015.01)
**A61P 25/28** (2006.01)

(21) Application number: **20166131.1**

(22) Date of filing: **27.03.2020**

(52) Cooperative Patent Classification (CPC):
**A61K 35/12; A61K 35/28; A61P 25/28**

(54) **ISOLATED MITOCHONDRIA FOR USE IN THE TREATMENT AND/OR PREVENTION OF ALZHEIMER S DISEASE**

ISOLIERTE MITOCHONDRIEN ZUR VERWENDUNG BEI DER BEHANDLUNG UND/ODER PRÄVENTION VON MORBUS ALZHEIMER

MITOCHONDRIES ISOLÉES À UTILISER DANS LE TRAITEMENT ET/OU LA PRÉVENTION DE LA MALADIE D'ALZHEIMER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.03.2019 TW 108110798**

(43) Date of publication of application:
**30.09.2020 Bulletin 2020/40**

(73) Proprietor: **Taiwan Mitochondrion Applied Technology Co. Ltd.**
**Zhubei City 302 (TW)**

(72) Inventors:
• **CHENG, Han-Chung**
**302 Zhubei City, Hsinchu County (TW)**
• **TU, Chi-Tang**
**302 Zhubei City, Hsinchu County (TW)**
• **HSU, Chih-Kai**
**302 Zhubei City, Hsinchu County (TW)**

(74) Representative: **Vossius & Partner**
**Patentanwälte Rechtsanwälte mbB**
**P.O. Box 86 07 67**
**81634 München (DE)**

(56) References cited:
**WO-A1-2016/008937**    **WO-A1-2020/146874**
**US-A1- 2008 275 005**    **US-A1- 2011 008 310**

• **NITZAN KEREN ET AL: "Mitochondrial Transfer Ameliorates Cognitive Deficits, Neuronal Loss, and Gliosis in Alzheimer's Disease Mice.",** JOURNAL OF ALZHEIMER'S DISEASE : JAD 2019, vol. 72, no. 2, 2019, pages 587 - 604, XP009521197, ISSN: 1875-8908
• **SHI XIANXUN ET AL: "Intravenous administration of mitochondria for treating experimental Parkinson's disease",** MITOCHONDRION, ELSEVIER, AMSTERDAM, NL, vol. 34, 24 February 2017 (2017-02-24), pages 91 - 100, XP085002374, ISSN: 1567-7249, DOI: 10.1016/J.MITO.2017.02.005
• **FAIZI M ET AL: "A search for mitochondrial damage in Alzheimer s disease using isolated rat brain mitochondria",** IRANIAN JOURNAL OF PHARMACEUTICAL RESEARCH 20161201 IRANIAN JOURNAL OF PHARMACEUTICAL RESEARCH IRN, vol. 15, 1 December 2016 (2016-12-01), pages 185 - 195, XP009521196, ISSN: 1735-0328
• **ZAMBRANO KEVIN ET AL: "The war against Alzheimer, the mitochondrion strikes back!",** MITOCHONDRION, ELSEVIER, AMSTERDAM, NL, vol. 64, 23 March 2022 (2022-03-23), pages 125 - 135, XP087026483, ISSN: 1567-7249, [retrieved on 20220323], DOI: 10.1016/ J.MITO.2022.03.003

**Description**

**BACKGROUND OF THE INVENTION**

**CROSSED-REFERENCE TO RELATED APPLICATION**

This application claims priority to and the benefit of Taiwan Patent Application No. 108110798, filed on March 27, 2019, in the Taiwan Intellectual Property Office.

**1. FIELD OF THE INVENTION**

The present invention relates to isolated mitochondria for use in the treatment and/or prevention of Alzheimer's disease. The isolated mitochondria may improve memory deficits and/or learning impairments of a subject caused by Alzheimer's disease, and/or the isolated mitochondria may reduce the concentration of $\beta$-amyloid peptides in a cell of a subject.

**2. DESCRIPTION OF THE PRIOR ART**

Alzheimer's disease (AD) is the most common type of dementia. As the number of people with Alzheimer's worldwide continues to increase, and there is currently no effective treatment, it is one of the global concerns. There are currently more than 50 million Alzheimer's patients worldwide, and it is expected to increase to more than 130 million by 2050. At present, the cost of medical care for Alzheimer's disease worldwide is about 800 billion U.S. dollars, which is an enormous economic burden on society.

The exact cause of Alzheimer's disease is still unknown, but plaques and neurofibrillary tangles (NFTs) are observed in brain tissue from Alzheimer's disease patients. Plaques are the result of amyloid beta peptide (A$\beta$) aggregation in the brain. Current research suggests that the aggregation of $\beta$-amyloid peptides will lead to an increase in oxidative stress and neurotoxicity in the brain, which cause damage and necrosis of brain nerve cells. Therefore, the abnormal aggregation of $\beta$-amyloid peptides is considered to be the main cause of Alzheimer's disease.

Two types of drugs are currently used to relieve Alzheimer's disease, one is cholinesterase inhibitor, the other is N-methyl-D-aspartate (NMDA) receptor antagonist. Acetylcholine is an important neurotransmitter in the brain. Decreased acetylcholine concentration in patients with Alzheimer's disease can cause degeneration of the cerebral cortex and memory loss. Therefore, in patients with mild and moderate Alzheimer's disease, acetylcholinease inhibitors are often used to improve memory loss. Commonly used acetylcholine inhibitors include rivastigmine, donepezil, galantanime. However, the long-term follow-up studies show that the efficacy of these drugs is quite limited, and there are doubts about whether it can actually improve Alzheimer's disease. In addition, active NMDA receptors cause excitatory neurons, but overactivation of NMDA receptors can leads to excitotoxicity, by which nerve cells are damaged or killed. Therefore, NMDA receptor antagonists are used to block activation of NMDA receptors in order to stop excitotoxic neuronal death. NMDA receptor antagonists are also commonly used to treat patients with moderate and severe Alzheimer's disease. Memantine is the only drug of this class. However, in a 2011 study published by Schneider et al., there was no sufficient evidence to suggest that memantine could slow the treatment of Alzheimer's disease. Although many new therapies are now being introduced, such as stem cell therapy or gene therapy, these novel therapies are still in research stage and there are doubts about safety and carcinogenicity of these novel therapies.

Faizi M., et al., (2016) Iranian Journal of Pharmaceutical Research, 15:185-195 studies factors which may play a role in the initiation of AD.

US 2011/0008310 A1 discloses abnormalities in mitochondrial structures in brain tissue of bipolar disorder patients.

WO 2020/146874 A1 relates to the treatment of a disease using isolated fibroblasts.

**SUMMARY OF THE INVENTION**

The invention relates to the embodiments as characterized in the claims and as described herein below.

Accordingly, the present invention relates to isolated mitochondria for use in the treatment and/or prevention of Alzheimer's disease.

The isolated mitochondria for use in the treatment and/or prevention of Alzheimer's disease may improve memory deficits and/or learning impairments of a subject caused by Alzheimer's disease.

The isolated mitochondria for use in the treatment and/or prevention of Alzheimer's disease may reduce the concentration of $\beta$-amyloid peptides in a cell of a subject.

## BRIEF DESCRIPTION OF THE DRAWINGS

In the drawing:

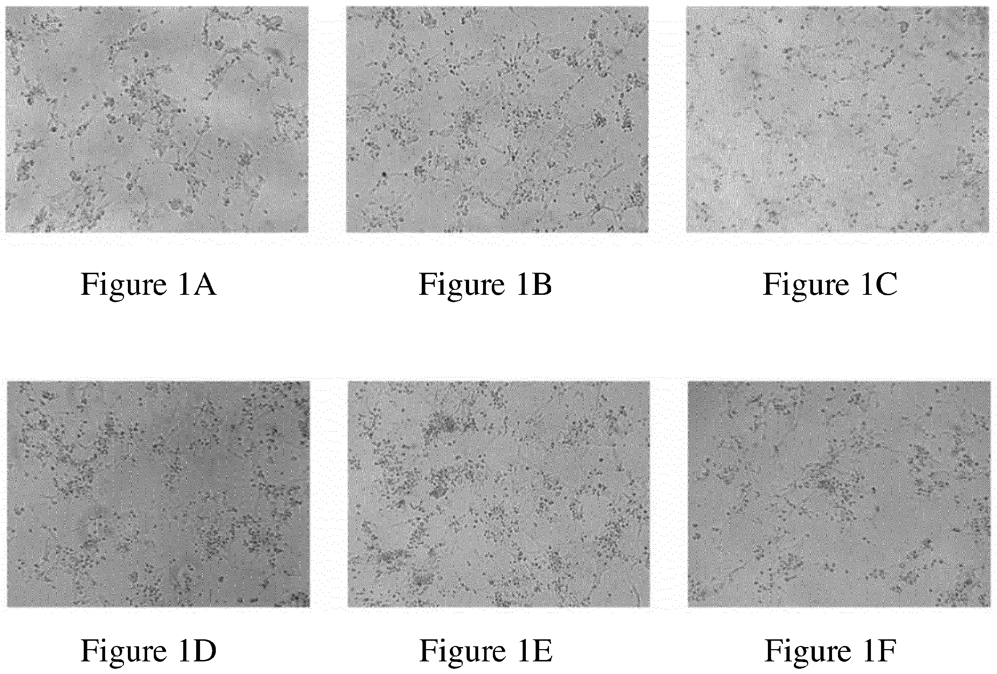

Figures 1A, 1B, and 1C show the cell morphology of neurons derived from induced pluripotent stem cells of patients with down syndrome (DS-Neurons) treated with 0 μg (control), 15 μg/ml, and 40 μg/ml of mitochondria, respectively, for 24 hours. Figures 1D, 1E, and 1F show the cell morphology of DS-Neurons treated with 0 μg (control), 15 μg/ml, and 40 μg/ml of mitochondria, respectively, for 48 hours.

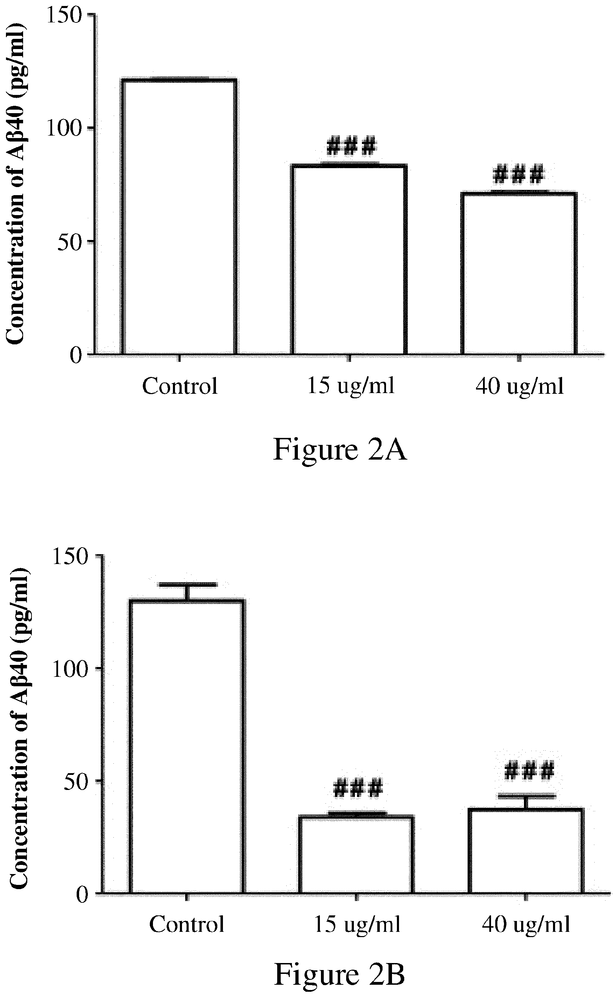

Figure 2A shows concentrations of amyloid beta peptide 40 (Aβ40) in DS-Neurons treated with 0 μg (control), 15 μg/ml, and 40 μg/ml, respectively, for 24 hours. Figure 2B shows concentrations of amyloid beta peptide 40 (Aβ40) in DS-Neurons treated with 0 μg (control), 15 μg/ml, and 40 μg/ml, respectively, for 48 hours. Data are represented as the mean ± standard error of mean (SEM). In comparison with the control group, ###, $p<0.001$.

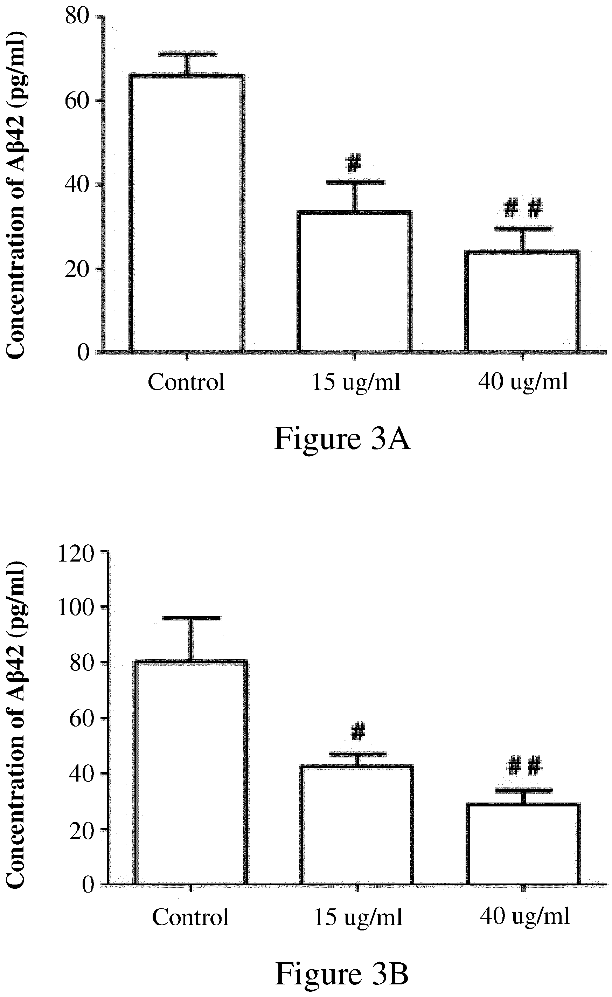

Figure 3A shows concentrations of amyloid beta peptide 42 (Aβ42) in DS-Neurons treated with 0 μg (control), 15 μg/ml, and 40 μg/ml, respectively, for 24 hours. Figure 3B shows concentrations of amyloid beta peptide 42 (Aβ42) in DS-Neurons treated with 0 μg (control), 15 μg/ml, and 40 μg/ml, respectively, for 48 hours. Data are represented as the mean ± SEM. In comparison with the control group, #, $p<0.05$; ##, $p<0.01$.

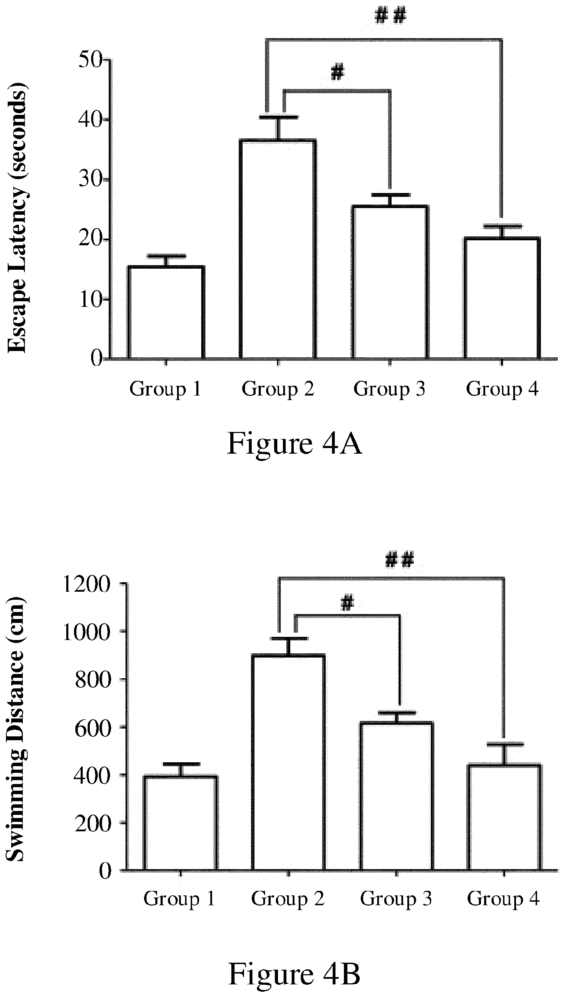

Figures 4A and 4B show the average escape latency and swimming distance of mice with different treatments in water maze tests, respectively. Mice were randomly divided into Group 1 (control), Group 2 (injection with β-amyloid peptide), Group 3 (injection with β-amyloid peptide and 30 μg mitochondria), and Group 4 (injection with β-amyloid peptide and 60 μg mitochondrion). Data are represented as the mean ± SEM. In comparison with the control group, #, $p<0.05$; ##, $p<0.01$.

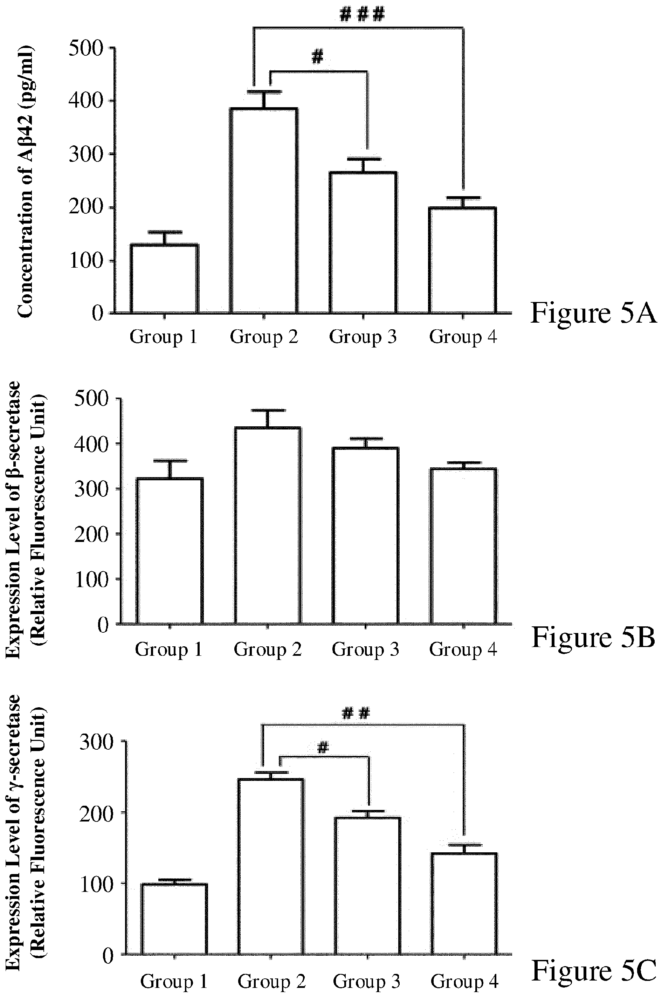

Figures 5A, 5B, and 5C show the expression levels of Aβ42, β-secretase, and γ-secretase in the brains of mice with different treatments, respectively. Mice were randomly divided into Group 1 (control), Group 2 (injection with β-amyloid peptide), Group 3 (injection with β-amyloid peptide and 30 μg mitochondria), and Group 4 (injection with β-amyloid peptide and 60 μg mitochondrion). Data are represented as the mean ± SEM. In comparison with the control group, #, $p<0.05$; ##, $p<0.01$; ###, $p<0.001$.

## DETAILED DESCRIPTION OF THE PREFERRED EMBODIMENT

The present invention provides isolated mitochondria for use in the treatment and/or prevention of Alzheimer's disease.

The "Guidance for Industry and Reviewers Estimating the Safe Starting Dose in Clinical Trials for Therapeutics in Adult Healthy Volunteers" published by the U.S. Department of Health and Human Services Food and Drug Administration discloses "a human equivalent dose (HED)" may be obtained by calculations from the following formula:

$$HED = \text{animal dose in mg/kg} \times (\text{animal weight in kg/human weight in kg})^{0.33}.$$

In some embodiments, the doses used in the mouse trials are 30 μg per mouse and 60 μg per mouse (a mouse weighs 30 mg), and the HEDs for a 60 kg human calculated are 0.0815 and 0.163 mg/kg, respectively.

In some embodiments, the mitochondria are to be administered to a subject, preferably a human, and the dosage is at least about 0.07 mg/kg, about 0.08 mg/kg, about 0.09 mg/kg, about 0.10 mg/kg, about 0.11 mg/kg, about 0.12 mg/kg, about 0.13 mg/kg, about 0.14 mg/kg, about 0.15 mg/kg, about 0.16 mg/kg, about 0.17 mg/kg, about 0.18 mg/kg, about 0.19 mg/kg, about 0.20 mg/kg, about 0.21 mg/kg, about 0.22 mg/kg, about 0.23 mg/kg, about 0.24 mg/kg, or about 0.25 mg/kg body weight.

In some embodiments, the isolated mitochondria are isolated exogenous mitochondria.

In some embodiments, the isolated mitochondria are derived from a stem cell. In some preferred embodiments, the isolated mitochondrial are derived from an adipose stem cell.

In some embodiments, the isolated mitochondria improve memory deficits and/or learning impairments caused by Alzheimer's disease.

In some embodiments, the isolated mitochondria are not encapsulated.

In some embodiments, the isolated mitochondria are to be administered to a subject by brain injection.

The invention further provides isolated mitochondria for use in the treatment and/or prevention of Alzheimer's disease, wherein the isolated mitochondria improve memory deficits and/or learning impairments of a subject caused by Alzheimer's disease.

The invention also provides isolated mitochondria for use in the treatment and/or prevention of Alzheimer's disease, wherein the isolated mitochondria reduce the concentration of β-amyloid peptides in a cell of a subject.

In some embodiments, the cell of the subject is a brain neural cell.

Unless otherwise defined, all technical and scientific terms used herein have the same meaning as commonly understood by one of ordinary skill in the art to which this invention pertains. In the case of conflict, the present document, including definitions will control.

As used herein, the term "isolated mitochondria" refers to the mitochondria that are separated from a cell in which they were originally present and maintained in a non-cellular environment that maintains their activity. In some embodiments, the isolated mitochondria of the present invention are in a buffer solution capable of maintaining the activity of the mitochondria. In some embodiments, the buffer solution contains a protease inhibitor.

As used herein, the term "isolated mitochondria are not encapsulated" refers to that the mitochondria that are separated from a cell in which they were originally present and maintained in a non-cellular environment that maintains their activity are not packaged by any drug delivery system. Drug delivery systems include, but are not limited to liposomes, lipid bilayer, lipid micelles, lipid raft, clathrin-coated vesicles.

As used herein, the term "aqueous buffer" or "buffer solution", which are used interchangeably, refers to an aqueous solution consisting of a mixture of a weak acid and its conjugate base, or a weak alkali and its conjugate base, which can reduce the effect of addition of a strong acid or a strong alkali on the pH of the solution to a certain extent, and therefore maintain the stability of the pH of the solution. In some embodiments, the buffer solution includes sucrose, ethylene glycol tetraacetic acid (EGTA), and N-(2-Hydroxyethyl) piperazine-N'-2-ethanesulfonic Acid (HEPES). In some embodiments, the buffer solution is SHE buffer, including 0.25 M sucrose, 0.5 mM EGTA, and 3 mM HEPES.

As used herein, the term "exogenous mitochondria" or "foreign mitochondria" refers to mitochondria that do not originate from the subject to whom the mitochondria are administered. Thus, "administering exogenous mitochondria to a subject" means administering to the subject mitochondria that are not from the subject themselves. Sources of exogenous mitochondria include, but are not limited to, another individual of the same species and an individual of different species (heterogeneous). For example, a human subject is administered with exogenous mitochondria, which may originate from a human who is not the subject, a group of humans not including the subject, or other species, such as mice, rats, rabbits, cattle, sheep, horses, monkeys, apes.

The isolated mitochondria for use in the present invention may reduce the concentration of $\beta$-amyloid peptides in a cell of a subject by administering to the subject an effective dose of isolated mitochondria, thereby treating and/or preventing Alzheimer's disease, and/or improving in memory deficits and/or learning impairments. $\beta$-amyloid peptides are produced through the proteolytic processing of a transmembrane protein, amyloid precursor protein (APP), by $\beta$-secretase, also known as $\beta$-site amyloid precursor-cleaving enzyme (BACE), and $\gamma$-secretase. Therefore, when the activities of $\beta$-secretase and $\gamma$-secretase in a tissue decrease, the concentration of $\beta$-amyloid peptides in the tissue reduces as well. $\beta$-amyloid peptides mainly exist in two forms, $\beta$-amyloid peptide 40 (A$\beta$40, with 40 amino acids) and $\beta$-amyloid peptide 42 (A$\beta$42, with 42 amino acids). A$\beta$42 is not easily broken down and easily aggregates outside nerve cells to form plaques. The $\beta$-amyloid peptide content in blood is a common indicator of early stage of Alzheimer's disease. Therefore, the content of A$\beta$40 and A$\beta$42 and the content of $\beta$-secretase and $\gamma$-secretase are often used as the basis and indicators for studying the formation of Alzheimer's disease and the efficacy of a drug.

As used herein, the term "reducing a concentration of $\beta$-amyloid peptides" refers to that the concentration of $\beta$-amyloid peptides in the brain of a subject treated with the mitochondria provided by the present invention is lower than the concentration of $\beta$-amyloid peptides in the brain of a subject not treated with the mitochondria provided by the present invention. Alternatively, the term refers to that the concentration of $\beta$-amyloid peptides in the brain of a subject after the subject was treated with the mitochondria provided by the present invention is lower than the concentration of $\beta$-amyloid peptides in the brain of the same subject before the subject was treated with the mitochondria provided by the present invention. In some embodiments, the term "reducing a concentration of $\beta$-amyloid peptides" refers to that a subject who was induced to have an abnormally high concentration of $\beta$-amyloid peptides has a reduced concentration of $\beta$-amyloid peptides after the subject was treated with isolated mitochondria, compared to another subject who had the same induction to have an abnormally high concentration of $\beta$-amyloid peptides but was not treated with isolated mitochondria.

As used herein, the term "neurons derived from induced pluripotent stem cells of patients with Down Syndrome (DS-Neurons)" refers to the neurons derived from Down Syndrome induced pluripotent stem cells (DS-iPSCs), which are induced from somatic cells of Down Syndrome (DS) patients. Down Syndrome is a genetic disorder caused by the presence of all or part of a third copy of chromosome 21. In humans, the gene for amyloid precursor protein (APP) is located on chromosome 21. Therefore, most DS patients have early onset major neurocognitive disorder and early onset Alzheimer's disease, and $\beta$-amyloid accumulation can be found in their brains, which make DS patients a research model for Alzheimer's disease. Down Syndrome induced pluripotent stem cells (DS-iPSCs), which are derived from somatic cells of Down Syndrome (DS) patients, have the ability to differentiate into neurons (DS-Neurons). DS-Neurons produce A$\beta$40 and A$\beta$42, which are important indicators of Alzheimer's disease, and induce aggregation of A$\beta$ and formation of plaques. Therefore, DS-Neurons can be used as a drug screening platform, in which the clearance of A$\beta$ is an important indicator of a potential drug for treating and/or delaying Alzheimer's disease. In some embodiments, the mitochondria provided by the present invention can effectively reduce $\beta$-amyloid aggregation in DS-Neurons and can be used for treating and/or delaying Alzheimer's disease.

As used herein, the term "pharmaceutical composition" refers to any formulation wherein the mitochondria of the invention may be formulated, stored, preserved, altered, administered, or a combination thereof. As described below, the formulation may comprise any pharmaceutically-acceptable diluent, adjuvant, buffer, excipient, carrier, or combination thereof. In general, components of the formulation are selected on the basis of the mode and route of administration, and standard pharmaceutical practice.

As used herein, the term "pharmaceutically acceptable carrier" means that any substance or combination thereof with the mitochondria of the present invention can be physically or chemically mixed, dissolved, suspended, or otherwise combined to yield the pharmaceutical composition of the present invention.

As used herein, the term "pharmaceutically effective amount" refers to an amount capable of or sufficient to maintain or produce a desired physiological result, including but not limited to treating, reducing, eliminating, substantially preventing, or prophylaxing, or a combination thereof, a disease, disorder, or combination thereof. A pharmaceutically effective amount may comprise one or more doses administered sequentially or simultaneously. Those skilled in the art will know to adjust doses of the present invention to account for various types of formulations, including but not limited to slow-release formulation.

As used herein, the terms "treat," "treating," "treatment" and "improve," "improving," "improvement", which are used interchangeably, refer to being able to cure, reduce, stop the progression, slow down the progression, advantageously change, eliminate, or a combination thereof, any aspect of a disease, condition, or combination thereof.

As used herein, the term "prevent," "preventing," or "prevention" refers to being able to substantially preclude, avert, obviate, forestall, stop, hinder, or a combination thereof, any aspect of a disease, condition, or combination thereof from happening, especially by advance action.

As used herein, the term "subject" refers to any individual to whom administration of the present invention is directed. A subject may be, for example, a mammal. The subject may be a human or veterinary animal, without regard to sex, age, or any combination thereof, and including fetuses. A subject may optionally be afflicted with, at risk for, or a combination thereof a particular disease, disorder, or combination thereof.

As used herein, the articles "a," "an," and "any" refer to one or more than one (i.e., at least one) of the grammatical object of the article. For example, "an element" means one element or more than one element.

As used herein, "around," "about," or "approximately" shall generally mean within 20 percent, preferably within 10 percent, and more preferably within 5 percent of a given value or range. Numerical quantities given herein are approximate, meaning that the term "around," "about," or "approximately" can be inferred if not expressly stated.

The present invention is further illustrated by the following examples, which are provided for the purpose of demonstration rather than limitation. Those skilled in the art should, in light of the present disclosure, appreciate that many changes can be made in the specific embodiments which are disclosed and still obtain a like or similar result.

# EXAMPLE

## Example 1 Preparation of Neurons Derived from Induced Pluripotent Stem Cells of Patients with Down Syndrome (DS-Neurons)

1. Cell Culture of Induced Pluripotent Stem Cells of Patients with Down Syndrome (DS-iPSCs)

DS-iPSCs (provided by Dr. Honglin Su, Department of Life Sciences, National Chung Hsing University, Taichung, Taiwan) were cultured in a serum-free medium, Essential 8™ (Life Technology, USA) and subjected to attachment culture with Matrigel™ (Becton-Dickinson, USA). Then, the culture medium was removed, and the cells were washed twice with Dulbecco's phosphate-buffered saline (DPBS, Corning, Cat. no. 21-031-CV, USA). After that, Accutase™ (Merck Millipore, USA) was added to the cells and reacted with the cells at 37°C for 2 to 5 minutes, and then the medium was added to stop the reaction. The cells were washed, dispersed, and centrifuged at 1,000 rpm for 2 minutes to remove the supernatant. The cells were then subcultured on a culture plate containing fresh serum-free medium, Essential 8™, for about 3 to 5 days, and the culture medium was changed every day.

2. Differentiation of Induced Pluripotent Stem Cells of Patients with Down Syndrome (DS-iPSCs) into Neurons

The above-mentioned DS-iPSCs were cultured to 80 to 90% confluency, and the cells were washed twice with DPBS. After that, Accutase™ (Merck Millipore, USA) was added to the cells and reacted with the cells at 37°C for 2 to 5 minutes, and then the medium was added to stop the reaction. The cells were washed, dispersed, and centrifuged at 800 rpm for about 2 minutes to remove the supernatant. The cells were then cultured in Dulbecco's Modified Eagle Medium: Nutrient Mixture F-12 (DMEM-F12) supplemented with 20% (v/v) knock out serum replacement (KSR, Life Techonolgy, USA) for 2 days to obtain suspension of embryonic bodies.

The suspended embryoid bodies were placed in a centrifuge tube, and after embryoid bodies precipitated naturally, the supernatant was removed. A neural induction medium [each 500 mL of the neural induction medium contains 326 mL of DMEM medium (Life Technologies, Cat. No. 11965-092, USA), 163 mL of F12 medium (Life Technologies, Cat. No. 11765-054, USA), 5 mL N-2 Supplement (Life Technologies, Cat. No. 17502-048, USA), 5 mL of non-essential amino acid (Life Technologies, Cat. No. 11140-035, USA), and 1 mL of heparin (1 mg/mL)] containing small-molecule drugs, BiSF [0.5 $\mu$M BIO (Sigma-Aldrich, USA), 10 ng/ml fibroblast growth factor-2 (FGF-2, Peprotech, USA), and 10 $\mu$M SB431542 (Sigma-Aldrich, USA)], was added to the cells for suspension culture for 2 days. Then, the suspended embryoid bodies exhibited a circular epithelial cell structure.

After the embryoid bodies precipitated, the supernatant was removed. A neural basal medium [each 521 mL of the neural induction medium contains 500 mL of neural basal medium (Life Technologies, Cat. No. 21103-049, USA), 5 mL of N-2 supplement (Life Technologies, Cat. No. 17502-048, USA), 5 mL of non-essential amino acid (Life Technologies, Cat. No. 11140-035, USA), 1 mL of Heparin (1 mg/mL), and 10 mL of B-27™ Supplement (Life Technologies, Cat. No. 17504-044, USA)] supplemented with 10 ng/ml fibroblast growth factor-2 (FGF-2) was added to the cells for suspension culture for 2 days. After the embryoid bodies precipitated, the embryoid bodies were dispersed with force or Accutase™, seeded on a culture dish coated with 1% (v/v) Matrigel™ [dissolved in DMEM/F12 medium (Gibco, Cat. No. 11330032, USA)] for cell attachment and culture. After about 2 to 7 days, the embryoid bodies differentiated into neurons, that is, neurons derived from induced pluripotent stem cells of patients with Down Syndrome (DS-Neurons).

3. Subculture of Neurons Derived from Induced Pluripotent Stem Cells of Patients with Down Syndrome (DS-Neurons)

The above-mentioned DS-Neurons were cultured to 80 to 90% confluency, and the cells were washed twice with DPBS. After that, Accutase™ was added to the cells and reacted with the cells at 37°C for 2 to 5 minutes, and then Neurobasal™ medium (Gibco, Cat. No. 21103049, USA) was added to stop the reaction. The cells were washed, dispersed, and centrifuged at 1,000 rpm for about 2 minutes to remove the supernatant. Neurobasal™ medium was added to adjust the cell concentration to $1 \times 10^5$ cells/mL, and the ROCK inhibitor Y-27632 (with a final concentration of 10 $\mu$M) (Merck Millipore Corporation, Cat. No. SCM075, USA) was added. The cells were then seeded on a culture dish coated with 1% (v/v) Matrigel™ (dissolved in DMEM/F12 medium), and Neurobasal™ medium was added for cell culture. On the next day, the Neurobasal™ medium was removed and replaced with Neurobasal™ medium containing 2% (v/v) B-27™ supplement and 10 ng/ml recombinant human Fibroblast Growth Factor (rh-FGF, Gibco, USA). Fresh culture medium was replaced every 2 to 3 days until the next passage.

**Example 2 Preparation of Mitochondria**

The mitochondria of this Example were isolated and purified from human adipose-derived stem cells (ADSCs). Human adipose-derived stem cells were cultured to $1 \times 10^8$ cells, rinsed with DPBS, and reacted with Accutase™ (Merck Millipore, USA) at 37 °C for 2 to 5 minutes. Then, the culture solution was added to stop the reaction, and the cells were washed, dispersed, and centrifuged at 1,000 rpm for about 2 minutes to remove the supernatant. The cells were resuspended with 2 ml of SHE buffer [0.25 M sucrose, 0.5 mM ethylene glycol tetraacetic acid (EGTA), 3 mM N-(2-hydroxyethyl) piperazine-N'-ethanesulfonic acid (HEPES), pH 7.2; all were purchased from Sigma-Aldrich] and then homogenized on ice 15 times with a homogenizer. After that, the cells were centrifuged at *1,000 xg* for 15 minutes, and the supernatant was transferred to another tube and centrifuged at 9,000 *xg* for 10 minutes. Then, the supernatant was removed, and the pellet (mitochondria) was resuspended with 50 $\mu$l SHE buffer containing a protease inhibitor and stored at 4 °C for further use.

**Example 3 Cell Experiments**

1. Cell Treatment

The DS-Neurons obtained in Example 1 were seeded into a 24-well cell culture plate at a density of $2 \times 10^5$ cells/well and cultured in Neurobasal™ medium containing 2% (v/v) B-27™ supplement for 16 to 24 hours. The mitochondria obtained in Example 2 were added to the culture medium at a concentration of 15 $\mu$g/ml or 40 $\mu$g/ml per well, and then the plate was centrifuged at 1,500 *xg* at 4 °C for 15 minutes to keep the mitochondria at the bottom of the plate. The DS-Neurons were co-cultured with the mitochondria in a 37 °C incubator for 24 or 48 hours. After that, cell morphology was observed under a microscope, and the culture medium in each well was collected. The collected cell culture was then centrifuged at 1,000 rpm for 5 minutes, and the supernatant was stored at -80 °C for further analysis.

2. Enzyme-Linked Immunoadsorbent Assay (ELISA)

The expression levels of Aβ40 and Aβ42 of the DS-Neurons were analyzed by ELISA. LEGEND MAX™ β-Amyloid x-40 ELISA Kit (BioLegend, USA) and LEGEND MAX™ β-Amyloid x-42 ELISA Kit (BioLegend, USA) were performed according to manufacturers' instructions. The supernatant obtained from the co-culture of the DS-Neurons and the isolated mitochondria mentioned above was diluted with incubation buffer (BioLegend, USA) at a ratio of 1:5. Fifty (50) μl of the diluted supernatant was added to the reaction plate provided by LEGEND MAX™ β-Amyloid x-40 ELISA Kit and/or LEGEND MAX™ β-Amyloid x-42 ELISA Kit. Each sample was repeat three times. Fifty (50) μl of horseradish peroxidase (HRP) labeled detection antibody (HRP detection antibody, BioLegend, USA) was added to the plates, and the plate was incubated at 4 °C for 16 hours. The mixture was removed, and the plate was washed with 300 μl wash buffer (BioLegend, USA) 5 times. After that, 200 μl 3,3',5,5'-tetramethylbenzidine (TMB substrate, BioLegend, USA) was added to the plate, and the plate was incubated at room temperature in the dark for 40 to 50 minutes. Then, the absorbance of the sample at 620 nm was measured with an ELISA reader, and the concentration of Aβ40 and Aβ42 in a sample was obtained by converting the standard curve of the standard into the absorbance of the sample.

3. Statistical Analysis

For all statistical analyses and comparisons of multiple groups, a GraphPad Prism Program (GraphPad, San Diego, CA, USA) using ANOVA followed by Tukey's *post hoc* test has been used. A $p < 0.05$ value was considered significant.

4. Results

Figures 1B, 1C, show the cell morphology of DS-Neurons treated with 15 μg/ml, and 40 μg/ml of mitochondria, respectively, for 24 hours. Figures 1E, 1F, show the cell morphology of DS-Neurons treated with 15 μg/ml, and 40 μg/ml of mitochondria, respectively, for 48 hours. The cell morphology of DS-Neurons without treatment with mitochondria (negative controls; Figuers 1A and 1D) and the cell morphology of DS-Neurons treated with mitochondria (Figure 1B, 1C, 1E, and 1F) have no difference. The results indicate that 15 μg/ml or 40 μg/ml of mitochondria cause no cytotoxicity to DS -Neurons.

As shown in Figures 2A and 2B, DS-Neurons treated with 15 μg/ml or 40 μg/ml for either 24 hours or 48 hours all have significantly lower concentrations of Aβ40 than DS-Neurons without treatment of mitochondria (control) (p<0.001). In addition, as shown in Figures 3A and 3B, DS-Neurons treated with 15 μg/ml or 40 μg/ml for either 24 hours or 48 hours all have significantly lower concentrations of Aβ42 than DS-Neurons without treatment of mitochondria (control) (p<0.05 or *p*<0.01). The results indicate that isolated mitochondria reduce the expression of β-amyloid peptides and can be further used in the treatment of Alzheimer's disease.

**Example 4 Animal Trials**

1. Treatment of Experimental animals

Twenty-four (24) male C57BL/6 mice (9-week old/25-30 g/mouse, purchased from BioLASCO Co., Ltd, Taiwan), which had never used in any experiments, were randomly divided into 4 groups, and 6 mice for each group. The mice in each group are treated respectively as follows.

Group 1: Each mouse was injected with phosphate buffer solution (PBS) into its lateral ventricle for 14 consecutive days; on the 15th day of the trail, each mouse was injected with SHE buffer into its lateral ventricle (control group, normal mice);

Group 2: Each mouse was injected with 300 pmol Aβ42 into its lateral ventricle every day for 14 consecutive days to cause memory deficits and/or learning impairments; on the 15th day of the trail, each mouse was injected with SHE buffer into its lateral ventricle (Aβ42-treated group, mice with Alzheimer's disease symptoms);

Group 3: Each mouse was injected with 300 pmol Aβ42 into its lateral ventricle every day for 14 consecutive days to cause memory deficits and/or learning impairments; on the 15th day of the trail, each mouse was injected with 30 μg of the isolated mitochondria obtained in Example 2 (Aβ42 + mito 30-treated group, mice with Alzheimer's disease symptoms treated with 30 μg isolated mitochondria); and

Group 4: Each mouse was injected with 300 pmol Aβ42 into its lateral ventricle every day for 14 consecutive days to cause memory deficits and/or learning impairments; on the 15th day of the trail, each mouse was injected with 60 μg of the isolated mitochondria obtained in Example 2 (Aβ42 + mito 60-treated group, mice with Alzheimer's disease symptoms treated with 60 μg isolated mitochondria).

On the 27th day of the trial, all of the mice were used for behavior experiments (water maze learning).

2. Water Maze Learning

A plastic circular pool 183 cm in diameter was filled with water (25 ± 2 °C). A circular platform was placed at a specific location from the edge of the pool and submerged below the water surface, for mice to stand and take a rest. Water was made cloudy by adding toxic-free dye. Mice have to learn by recognizing and memorizing distinctive visual cues set on the wall of the pool. For memory and spatial learning, mice were subjected to 3 trials per day, with one trial early in the morning, one trial at noon, and another in the late afternoon. The training procedure lasted 4 days, and a total of 12 trials were given. The mice were positioned at different starting points spaced equally around the perimeter of the pool in random order. They had 60 seconds to swim in the pool. If a mouse could not find the platform, it was guided to the platform and was allowed to remain there for 20 seconds. The time each mouse took to reach the platform was recorded as the escape latency. The distance each mouse swam to reach the platform was also recorded.

3. Biochemical Analysis

The mice in each group were sacrificed after the behavior experiments, and the expression levels of Aβ42, β-secretase, and γ-secretase were measured. The concentrations of Aβ42 were analyzed by ELISA as described in Example 3. The expression levels of β-secretase and γ-secretase were analyzed by the following methods.

Analysis of expression level of β-secretase. Fifty (50) μl of each sample protein (at a concentration between 25-200 μg/ml) and each serially diluted standard sample were added to 96-well plates. Forty-eight (48) μl of 2X reaction buffer and 2 μl of β-secretase substrate were added to each sample. The mixture was incubated at 37°C in dark for 1 to 2 hours. The expression levels of sample proteins were measure using a plate reader with an excitation wavelength of 335 to 355 nm and an emission wavelength of 495 to 510 nm. The expression level (relative fluorescence unit, RFU) of β-secretase in a sample was obtained by converting the standard curve of the serially diluted standard samples.

Analysis of expression level of γ-secretase. One hundred (100) μl of each sample protein and each serially diluted standard sample were added to 96-well plates and incubated at 37°C for 2 hours. After that, samples were removed, 100 μl of biotin-antibody was added to each well and incubated at 37°C for 1 hour. Then, the antibody was removed, each well was rinsed with wash buffer 5 times, and 90 μl of 3,3',5,5'-Tetramethylbenzidine (TMB) substrate was added to each well and incubated at 37°C in dark for 15 to 30 minutes. Finally, 50 μl of stop solution was added to each well, and the absorbance of a sample at 450 nm was measured with a fluorescence reader. The expression level (RFU) of γ-secretase in a sample was obtained by converting the standard curve of the serially diluted standard samples.

4. Statistical Analysis

For all statistical analyses and comparisons of multiple groups, a GraphPad Prism Program (GraphPad, San Diego, CA, USA) using ANOVA followed by Tukey's *post hoc* test has been used. A $p < 0.05$ value was considered significant.

5. Results

5.1 *Isolated mitochondria improve spatial learning impairments and memory deficits caused by β-amyloid peptides.*

In this Example, animal model with learning impairments and memory deficits caused by β-amyloid peptides was used to study the effect of isolated mitochondria on Alzheimer's disease. As shown in Figures 4A and 4B, mice which were injected with Aβ42 only (Group 2) spend the longest time and swam the longest distance to reach the platform. Figure 4A also shows that mice which were injected with Aβ42 and followed by treatment of 30 μg or 60 μg of the isolated mitochondria (Group 3 or Group 4) had significantly less escape latency than mice of Group 2 ($p<0.05$ or $p<0.01$), and the isolated mitochondria improved spatial learning and memory of mice in a dose-dependent fashion. In addition, Figure 4B shows that mice which were injected with Aβ42 and followed by treatment of 30 μg or 60 μg of the isolated mitochondria (Group 3 or Group 4) swam significantly shorter distance than mice of Group 2 ($p<0.05$ or $p<0.01$). The results indicates that the isolated mitochondria improved spatial learning impairments and memory deficits of mice caused by β-amyloid peptides in a dose-dependent fashion.

5.2 *Administration of isolated mitochondria significantly reduce the expression level of Aβ42 and γ-secretase in the brain of mice.*

The effects of isolated mitochondria on the expression levels of Aβ42, β-secretase, and γ-secretase in the brains

8

of mice were further analyzed in this Example. As shown in Figures 5A, 5B, and 5C, mice which were injected with Aβ42 only (Group 2) had the highest expression levels of Aβ42, β-secretase, and γ-secretase. Figures 5A and 5C also show that mice which were injected with Aβ42 and followed by treatment of 30 μg or 60 μg of the isolated mitochondria (Group 3 or Group 4) had significantly less expression levels of Aβ42 and γ-secretase than mice of Group 2 ($p<0.05$, $p<0.01$, or $p<0.001$), and the isolated mitochondria reduce the expression level of Aβ42 and γ-secretase in the brain of mice in a dose-dependent fashion.

Because β-secretase and γ-secretase play important roles in the formation of β-amyloid peptides, and the aggregation of β-amyloid peptides is considered to be the main cause of Alzheimer's disease, the above results indicate that isolated mitochondria reduce the formation and aggregation of β-amyloid peptides in the brains of mice, and further improve the symptoms (memory deficits and learning impairments) of Alzheimer's disease in a dose-dependent fashion.

## Claims

1. Isolated mitochondria for use in the treatment and/or prevention of Alzheimer's disease.

2. Isolated mitochondria for use in the treatment and/or prevention of Alzheimer's disease according to claim 1, wherein the isolated mitochondria are derived from a stem cell.

3. Isolated mitochondria for use in the treatment and/or prevention of Alzheimer's disease according to claim 1, wherein an effective dose of the isolated mitochondria is at least 0.07 mg/kg of body weight of a subject.

4. Isolated mitochondria for use in the treatment and/or prevention of Alzheimer's disease according to claim 1, wherein the isolated mitochondria improve memory deficits and/or learning impairments caused by Alzheimer's disease.

5. Isolated mitochondria for use in the treatment and/or prevention of Alzheimer's disease according to claim 1, wherein the isolated mitochondria reduce the concentration of β-amyloid peptides in a cell of a subject.

## Patentansprüche

1. Isolierte Mitochondrien zur Verwendung bei der Behandlung und/oder Vorbeugung der Alzheimer-Krankheit.

2. Isolierte Mitochondrien zur Verwendung bei der Behandlung und/oder Vorbeugung der Alzheimer-Krankheit nach Anspruch 1, wobei die isolierten Mitochondrien von einer Stammzelle stammen.

3. Isolierte Mitochondrien zur Verwendung bei der Behandlung und/oder Vorbeugung der Alzheimer-Krankheit nach Anspruch 1, wobei eine wirksame Dosis der isolierten Mitochondrien mindestens 0,07 mg/kg Körpergewicht eines Individuums beträgt.

4. Isolierte Mitochondrien zur Verwendung bei der Behandlung und/oder Vorbeugung der Alzheimer-Krankheit nach Anspruch 1, wobei die isolierten Mitochondrien durch die Alzheimer-Krankheit hervorgerufene Gedächtnisdefizite und/oder Lernbeeinträchtigungen verbessern.

5. Isolierte Mitochondrien zur Verwendung bei der Behandlung und/oder Vorbeugung der Alzheimer-Krankheit nach Anspruch 1, wobei die isolierten Mitochondrien die Konzentration von β-Amyloid-Peptiden in einer Zelle eines Individuums verringern.

## Revendications

1. Mitochondries isolées pour une utilisation dans le traitement et/ou la prévention de la maladie d'Alzheimer.

2. Mitochondries isolées pour une utilisation dans le traitement et/ou la prévention de la maladie d'Alzheimer selon la revendication 1, lesquelles mitochondries isolées dérivent d'une cellule souche.

3. Mitochondries isolées pour une utilisation dans le traitement et/ou la prévention de la maladie d'Alzheimer selon la revendication 1, dans lesquelles la dose efficace des mitochondries isolées est d'au moins 0,07 mg/kg de poids corporel d'un sujet.

4. Mitochondries isolées pour une utilisation dans le traitement et/ou la prévention de la maladie d'Alzheimer selon la revendication 1, lesquelles mitochondries isolées améliorent les déficits mémoriels et/ou les troubles d'apprentissage causés par la maladie d'Alzheimer.

5. Mitochondries isolées pour une utilisation dans le traitement et/ou la prévention de la maladie d'Alzheimer selon la revendication 1, lesquelles mitochondries isolées réduisent la concentration de peptides β-amyloïdes dans une cellule d'un sujet.

Figure 1A

Figure 1B

Figure 1C

Figure 1D

Figure 1E

Figure 1F

Figure 2A

Figure 2B

Figure 3A

Figure 3B

Figure 4A

Figure 4B

Figure 5A

Figure 5B

Figure 5C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- TW 108110798
- US 20110008310 A1
- WO 2020146874 A1

### Non-patent literature cited in the description

- **FAIZI M. et al.** *Iranian Journal of Pharmaceutical Research*, 2016, vol. 15, 185-195
- Guidance for Industry and Reviewers Estimating the Safe Starting Dose. Clinical Trials for Therapeutics in Adult Healthy Volunteers. U.S. Department of Health and Human Services Food